# EUROPÄISCHE PATENTSCHRIFT

(11) **EP 0 546 422 B1**
(45) Veröffentlichungstag und Bekanntmachung des Hinweises auf die Patenterteilung: **26.07.1995**
(21) Anmeldenummer: 92120439.2
(22) Anmeldetag: 30.11.1992
(51) Int. Cl.: C07D 303/22, C07D 305/06, C08F 16/26, C08F 216/14

(54) **Verfahren zur Herstellung von Octadienylethern**
Process for the preparation of octadienylethers
Procédé pour la préparation d'octadiényl-éthers

(30) Priorität: 11.12.1991 DE 4140735
(43) Veröffentlichungstag der Anmeldung: 16.06.1993
(73) Patentinhaber: BAYER AG, 51368 Leverkusen (DE)
(72) Erfinder: Beitzke, Bernhard, Dr., W-5064 Rösrath-Forsbach (DE); Joentgen, Winfried, Dr., W-5000 Köln 71 (DE); Traenckner, Hans-Joachim, Dr., W-5090 Leverkusen 1 (DE); Wüst, Alfredo, Dr., W-5000 Köln 80 (DE)

(56) Entgegenhaltungen:
- EP-A- 0 378 863
- GB-A- 2 114 974
- CHEMICAL ABSTRACTS, vol. 86, no. 20, 16. Mai 1977, Columbus, Ohio, US; abstract no. 141793y, Seite 98 ;
- CHEMICAL ABSTRACTS, vol. 109, no. 25, 19. Dezember 1988, Columbus, Ohio, US; abstract no. 231376n, L.I. ZAKHARKIN ET AL. 'Synthesis of 6-(Z-2 ,7-octadienyl)-alpha-D-galactopyranose' Seite 899 ;
- CHEMICAL ABSTRACTS, vol. 93, no. 19, 10. November 1980, Columbus, Ohio, US; abstract no. 185682k, U.M. DZHEMILEV ET AL. 'Telomerization of polyhydric alcohols with butadiene catalyzed by low-valence palladium complexes' Seite 602 ;
- R.T. Morrison et al., Lehrbuch der Organischen Chemie, 3. Auflage, s. 1209-1214
- Ullmann's Encyclopedia of Industrial Chemistry, 5. Auflage, Band A9, s. 531-533

## Beschreibung

Die vorliegende Erfindung betrifft ein Verfahren zur Herstellung von Octadienylethern aus cyclischen Hydroxyethern und 1,3-Butadien.

Es ist bekannt, daß man durch Telomerisation aus Alkoholen mit konjugierten Dienen Octadienylether herstellen kann (siehe A.Behr, "Telomerisation of Dienes by Homogeneous Transition Metal Catalysis" in Aspects of Homogeneous Catalysis, Vol. 5, herausgegeben von R. Ugo (1984)). In ein solches Verfahren statt Alkoholen cyclische Hydroxyether einzusetzen hat nicht nahegelegen, weil unter den Reaktionsbedingungen mit der Spaltung von Etherbrücken zu rechnen war.

Es ist auch bekannt, Glycidol-2,7-octadien-1-yl-ether in einem 2-Stufen-Verfahren herzustellen, bei dem zunächst 2,7-Octadienol mit Epichlorhydrin unter saurer Katalyse zum entsprechenden Chlorhydrinether umgesetzt und dieser in Gegenwart von Natriumhydroxid oder Natriumaluminat zyklisiert wird (siehe JP-AS 76-40053). Nachteilig hierbei ist, daß 2,7-Octadienol nicht so gut zuganglich ist wie Butadien, die Durchführung der Reaktion in 2 Stufen (sauer und alkalisch) erfolgt und die Toxizität des Epichlorhydrins, das zu seiner Handhabung besonderen sicherheitstechnischen Aufwand verlangt.

Die GB-A-2 114 974 beschreibt u.a. die Umsetzung von Butan-, Pentan- und Hexandiol mit Budadien zu Octadienylhydroxyalkylethern, die keine Epoxygruppierung enthalten.

Aus C.A. 109, 231376n geht hervor, daß man 1,2,3,4-Di-O-isopropyliden-α-D-galactopyranose mit Budatien zu einem Octylether umsetzen kann.

C.A. 93, 185682k berichtet über die Umsetzung bestimmter Alkohole mit Butadien in Gegenwart einer Palladiumverbindung, einer Phosphorverbindung und Aluminiumtriethyl.

In der EP-A-378 863 werden Oxetanylgruppen enthaltende thermoplastische Polymerisate beschrieben, bei deren Herstellung Oxetanylgruppen enthaltende Monomere eingesetzt werden. Über die Herstellung Oxetanylgruppen enthaltender Monomere wird nichts ausgesagt.

Es wurde nun ein Verfahren zur Herstellung von Octadienylethern der Formel (I) 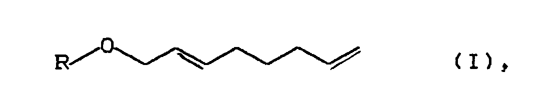
in der R für einen Rest der Formel 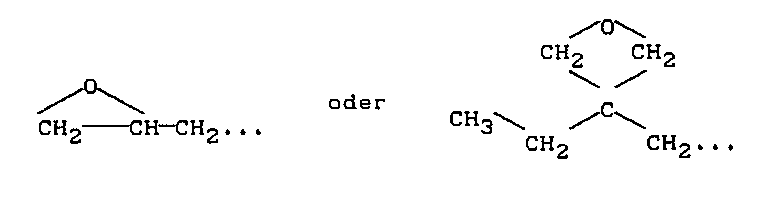
steht gefunden, das dadurch gekennzeichnet ist, daß man einen cyclischen Hydroxyether der Formel (II)

R-OH (II)

in der R die oben angegebene Bedeutung hat,
mit 1,3-Butadien in Gegenwart eines Palladiumkatalysators und eines phosphororganischen Cokatalysators bei 25 bis 100°C und einen Druck von 1 bis 20 bar umsetzt.

Bei den erfindungsgemäß einzusetzenden cyclischen Hydroxyethern handelt es sich um Glycidol und 3-Ethyl-3-hydroxymethyl-oxetan, die beide Handelsprodukte sind. Sie können in handelsüblichen Reinheiten eingesetzt werden. 1,3-Butadien kann ebenfalls in handelsüblichen Reinheiten eingesetzt werden.

Das Molverhältnis von 1,3-Butadien zum cyclischen Hydroxyether der Formel (II) kann beispielsweise 1,4 bis 2,2 : 1 betragen. Beim Einsatz von Glycidol beträgt dieses Verhältnis vorzugsweise 1,4 bis 1,8 : 1, beim Einsatz von 3-Ethyl-3-hydroxymethyl-oxiran vorzugsweise 1,7 bis 2,2 : 1.

Als Palladiumkatalysatoren kommen beispielsweise Pd(0)-und Pd(II)-Verbindungen in Frage. Bevorzugt sind Pd(II)-Verbindungen wie Palladium(II)-acetat und Palladium(II)-acetylacetonat, insbesondere Palladium(II)-acetylacetonat.

Als phosphororganische Cokatalysatoren kommen beispielsweise Triarylphosphane und Triarylphosphite in Frage. Bevorzugt ist Triphenylphosphan.

Der Palladiumkatalysator kann beispielsweise in Mengen von 10⁻⁶ bis 10⁻³ Mol Pro Mol cyclischen Hydroxyether der Formel (II), der phosphororganische Cokatalysator beispielsweise in Mengen von 1 bis 10 Mol pro Mol Palladiumkatalysator eingesetzt werden.

Bevorzugte Reaktionstemperaturen sind solche im Bereich 40 bis 85°C und bevorzugte Drucke solche im Bereich 3 bis 12 bar. Besonders bevorzugt ist die Zudosierung von 1,3-Butadien bei einem konstanten Druck im Bereich von 3 bis 12 bar.

Das erfindungsgemäße Verfahren kann in Abwesenheit oder Anwesenheit von Lösungsmitteln durchgeführt werden. Als Lösungsmittel kommen beispielsweise niedere sekundäre und tertiäre Alkohole und niedere Ketone in Frage, wie Isopropylalkohol, tert-Butylalkohol, Aceton, Methylethylketon und Methylisobutylketon. Bevorzugt werden jedoch keine Lösungsmittel zugesetzt.

Eine besonders bevorzugte Ausführungsform des erfindungsgemäßen Verfahrens wird wie folgt durchgeführt:
Der cyclischer Hydroxyether der Formel (II), Palladium(II)-acetylacetonat und Triphenylphosphan werden in einer mit Stickstoff gespülten druckfesten Apparatur vorgelegt. Dann wird bei Raumtemperatur 5 bis 15 Gew.-% der Gesamtmenge an 1,3-Butadien zugegeben, das Gemisch auf die Reaktionstemperatur erwärmt und das restliche Butadien bei konstantem Druck nachdosiert. Nach Beendigung der Butadienzugabe wird noch 1 bis 5 Stunden bei Reaktionstemperatur nachgerührt, danach auf Raumtemperatur abgekühlt und gegebenenfalls vorhandenes überschüssiges 1,3-Butadien durch Einleiten von Stickstoff entfernt. Dann destilliert man das Reaktionsgemisch, gegebenenfalls nach Zusatz eines Antioxidants (besonders vorteilhaft beim Destillieren von Reaktionsgemischen aus der Umsetzung von 3-Ethyl-3-hydroxymethyloxetan), im Vakuum. Das so erhaltene Rohprodukt kann z.B durch nochmalige Destillation im Vakuum weitergereinigt werden.

Das erfindungsgemäße Verfahren gestattet die Herstellung von Octadienylethern der Formel (I) auf technisch einfache Weise, in einem 1-Stufen-Verfahren, aus gut zugänglichen Ausgangsprodukten, in guten Ausbeuten und Selektivitäten und unter Vermeidung besonders toxischer Chemikalien. Überraschenderweise treten Spaltungen von Etherbrücken praktisch nicht auf.

Die erfindungsgemäß hergestellten Octadienylether der Formel (I) können als Monomerbaustein bei der Herstellung von Polyestern und Harzen verwendet werden. Sie stellen auch interessante Zwischenprodukte für die organische Synthese dar, beispielsweise zur Herstellung von Riechstoffen, Pharmazeutika und Pflanzenschutzmitteln.

Insbesondere kann man durch Copolymerisation von 3-Ethyl-3-[(2,7-octadienyloxy)methyl]-oxetan mit harzbildenden Monomeren wie Acrylnitril, Styrol oder Methylstyrol oxetangruppenhaltige Harze herstellen, die als Modifikatoren für PVC-Polymere eingesetzt werden können. PVC-Blends, die unter Verwendung dieser oxetangruppenhaltigen Modifikatoren hergestellt worden sind, zeigen eine höhere Wärmeformbeständigkeit und Thermostabilität bei der Verarbeitung und Temperaturlagerung im Vergleich zu den PVC-Blends, die mit Modifikatoren ohne Oxetangruppen hergestellt wurden.

### Beispiel 1

Ein Gemisch aus 1160 g 3-Ethyl-3-hydroxymethyl-oxetan, 1,5 g Palladium(II)-acetylacetonat und 10 g Triphenylphosphan wurde in einer mit Stickstoff gespülten, druckfesten Apparatur vorgelegt. Dann wurden bei Raumtemperatur 110 g 1,3-Butadien zugegeben, dann das Gemisch auf 70°C erwärmt. Bei dieser Temperatur wurden im Verlauf von 7 Stunden 980 g 1,3-Butadien zudosiert. Danach wurde noch 3 Stunden bei 70°C nachgerührt. Nicht umgesetztes Butadien wurde durch Einleiten von Stickstoff ausgetrieben. Das Rohgemisch wurde unter Zusatz von 0,5 Gew.-% 2,5-Ditertiärbutylhydrochinon im Vakuum fraktioniert destilliert. Bei 79 bis 81°C und 0,4 mbar ging ein 99 Gew.-% reines 3-Ethyl-3-[(2,7-octadienyloxy)methyl]-oxetan als Hauptprodukt über. Der Umsatz an 3-Ethyl-3-hydroxymethyl-oxetan betrug 74 %. Der gewünschte Ether wurde mit einer Selektivität von 98 % gebildet. Der Umsatz an 1,3-Butadien betrug etwa 80 %. In einer Ausbeute von ca. 7 % hatte sich durch Dimerisation von 1,3-Butadien als Nebenprodukt Octatrien gebildet.

Das IR-Spektrum von 3-Ethyl-3-[(2,7-Octadienyloxy)methyl]-oxetan (Film) wies folgende starke Banden auf (Angabe in cm⁻¹): 2930, 2860, 1460, 1105, 980 und 910.

Das ¹H-NMR-Spektrum von 3-Ethyl-3-[(2,7-Octadienyloxy)methyl]-oxetan (270 MHz; CDCl₃) wies folgende Signale auf (Angabe in ppm):
0,90 Triplett,
1,50 Multiplett,
1,75 Multiplett,
2,10 Multiplett,
3,55 Singulett,
3,95 Dublett,
4,40 Doppeldublett,
5,00 Multiplett,
5,50 Multiplett und
5,80 Multiplett.

### Beispiel 2

Ein Gemisch aus 670 g Glycidol, 0,15 g Palladium(II)-acetylacetonat und 1285 g Triphenylphosphan wurde in einer mit Stickstoff gespülten, druckfesten Apparatur vorgelegt. Dann wurden bei Raumtemperatur 110 g 1,3-Butadien hinzugefügt und anschließend das Gemisch auf 70°C erwärmt. Bei dieser Temperatur wurden im Verlauf von 7 Stunden 990 g 1,3-Butadien zudosiert. Anschließend wurde 3 Stunden lang bei 70°C nachgerührt, dann nicht umgesetztes Butadien durch Einleiten von Stickstoff ausgetrieben. Der danach verbliebene Rückstand wurde gaschromatisch untersucht.

Bei einem Glycidolumsatz von 99 % wurden 1,3-Butadien-Telomerisate des Glycidols mit einer Selektivität von 69 % erhalten.

### Beispiel 3

Ein Gemisch aus 800 g Glycidol, 0,055 g Palladium(II)-acetylacetonat und 0,470 g Triphenylphosphan wurde in einer mit Stickstoff gespülten, druckfesten Apparatur vorgelegt. Danach wurden bei Raumtemperatur 81 g 1,3-Butadien zugefügt und anschließend das Gemisch auf 70°C erwärmt. Bei dieser Temperatur wurden im Verlauf von 7 Stunden 730 g 1,3-Butadien zudosiert und anschließend bei 70°C 3 Stunden nachgerührt. Nicht umgesetztes Butadien wurde durch Einleiten von Stickstoff ausgetrieben. Der Rückstand wurde gaschromatografisch untersucht.

Bei einem Glycidolumsatz von 81 % wurden Butadien-Telomerisate des Glycidols mit einer Selektivität von 90 % erhalten.

## Patentansprüche

1. Verfahren zur Herstellung von Octadienylethern der Formel (I) 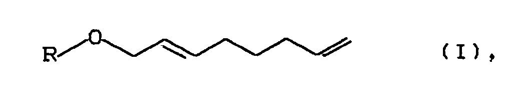 in der R für einen Rest der Formel 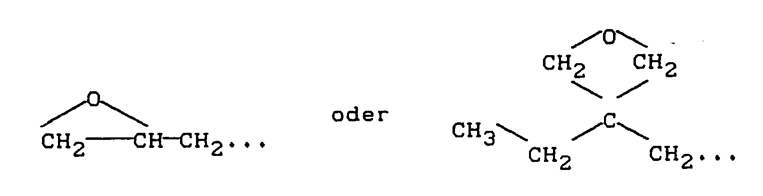 steht,
dadurch gekennzeichnet, daß man einen cyclischen Hydroxyether der Formel (II)
R-OH (II)
in der R die oben angegebene Bedeutung hat,
mit 1,3-Butadien in Gegenwart eines Palladiumkatalysators und eines phosphororganischen Cokatalysators bei 25 bis 100°C und einem Druck von 1 bis 20 bar umsetzt.

2. Verfahren nach Anspruch 1, dadurch gekennzeichnet, daß das Molverhältnis von 1,3-Butadien zum Alkohol der Formel (II) 1,4 bis 2,2 : 1 beträgt.

3. Verfahren nach Ansprüchen 1 und 2, dadurch gekennzeichnet, daß als Palladiumkatalysatoren Pd(0)-oder Pd(II)-Verbindungen und als phosphororganische Cokatalysatoren Triarylphosphane oder Triarylphosphite eingesetzt werden.

4. Verfahren nach Ansprüchen 1 bis 3, dadurch gekennzeichnet, daß der Palladiumkatalysator in Mengen von 10⁻⁶ bis 10⁻³ Mol pro Mol cyclischen Hydroxyether der Formel (II) und der phosphororganische Cokatalysator in Mengen von 1 bis 10 Mol pro Mol Palladiumkatalysator eingesetzt werden.

5. Verfahren nach Ansprüchen 1 bis 4, dadurch gekennzeichnet, daß man es bei Temperaturen im Bereich 40 bis 85°C und Drucken im Bereich von 3 bis 12 bar durchführt.

6. Verfahren nach Ansprüchen 1 bis 5, dadurch gekennzeichnet, daß man es ohne Zusatz von Lösungsmitteln durchführt.

## Claims

1. Process for the preparation of octadienyl ethers of the formula (I) 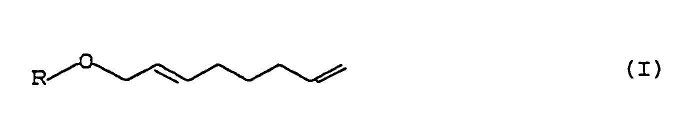 in which R represents a radical of the formula 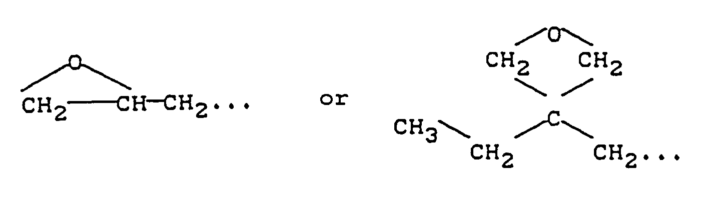 characterised in that a cyclic hydroxy-ether of the formula (II)
R-OH (II)
in which R has the abovementioned meaning,
is reacted with 1,3-butadiene in the presence of a palladium catalyst and an organophosphorus cocatalyst at 25 to 100°C under a pressure of 1 to 20 bar.

2. Process according to Claim 1, characterised in that the molar ratio of 1,3-butadiene to the alcohol of the formula (II) is 1.4 to 2.2:1.

3. Process according to Claims 1 and 2, characterised in that Pd(0) compounds or Pd(II) compounds are employed as the palladium catalysts and triarylphosphanes or triaryl phosphites are employed as the organophosphorus cocatalysts.

4. Process according to Claims 1 to 3, characterised in that the palladium catalyst is employed in amounts of 10⁻⁶ to 10⁻³ mol per mol of cyclic hydroxy-ether of the formula (II) and the organophosphorus cocatalyst is employed in amounts of 1 to 10 mol per mol of palladium catalyst.

5. Process according to Claims 1 to 4, characterised in that it is carried out at temperatures in the range from 40 to 85°C under a pressure in the range from 3 to 12 bar.

6. Process according to Claims 1 to 5, characterised in that it is carried out without addition of solvents.

## Revendications

1. Procédé de préparation d'éthers octadiényliques de formule (I) : 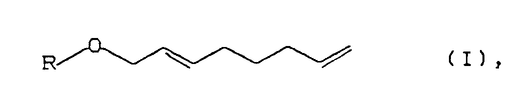 dans laquelle R représente un groupe de formule 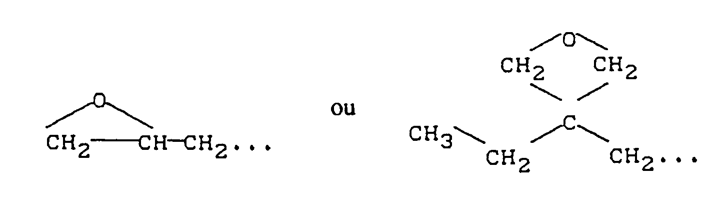 caractérisé en ce que l'on fait réagir un hydroxyéther cyclique de formule (II) :
R-OH (II)
dans laquelle R a les significations indiquées ci-dessus,
avec le 1,3-butadiène en présence d'un catalyseur au palladium et d'un catalyseur auxiliaire consistant en un dérivé organique du phosphore à des températures de 25 à 100°C sous une pression de 1 à 20 bars.

2. Procédé selon la revendication 1, caractérisé en ce que le rapport molaire entre le 1,3-butadiène et l'alcool de formule (II) est de 1,4 à 2,2:1.

3. Procédé selon les revendications 1 et 2, caractérisé en ce que l'on utilise en tant que catalyseurs au palladium des dérivés du Pd(0) ou du Pd(II) et en tant que catalyseurs auxiliaires consistant en dérivés organiques du phosphore des triarylphosphanes ou des phosphites de triaryle.

4. Procédé selon les revendications 1 à 3, caractérisé en ce que l'on met en oeuvre le catalyseur au palladium en quantité de 10⁻⁶ à 10⁻³ mol par mole de l'hydroxyéther cyclique de formule (II) et le catalyseur auxiliaire consistant en dérivé organique du phosphore en quantité de 1 à 10 mol par mole du catalyseur au palladium.

5. Procédé selon les revendications 1 à 4, caractérisé en ce que l'on opère à des températures de 40 à 85°C et des pressions de 3 à 12 bars.

6. Procédé selon les revendications 1 à 5, caractérisé en ce que l'on opère en l'absence de solvants.
